# EUROPÄISCHE PATENTSCHRIFT

(11) **EP 0 561 213 B1**
(45) Veröffentlichungstag und Bekanntmachung des Hinweises auf die Patenterteilung: **25.10.1995**
(21) Anmeldenummer: 93103363.3
(22) Anmeldetag: 03.03.1993
(51) Int. Cl.: C07C 69/675, C07C 67/44

(54) **Verfahren zur Herstellung von Hydroxypivalinsäureneopentylglykolester**
Process for the production of neopentylglycol hydroxypivalate
Procédé de fabrication d'hydroxypivalate du néopentylglycole

(30) Priorität: 18.03.1992 DE 4208571
(43) Veröffentlichungstag der Anmeldung: 22.09.1993
(73) Patentinhaber: BASF Aktiengesellschaft, 67063 Ludwigshafen (DE)
(72) Erfinder: Merger, Franz, Dr., W-6710 Frankenthal (DE); Schmidt-Radde, Martin, Dr., W-6711 Beindersheim (DE)

(56) Entgegenhaltungen:
- EP-A- 0 005 471
- DE-A- 2 234 110

## Beschreibung

Die vorliegende Erfindung betrifft ein verbessertes Verfahren zur Herstellung von Hydroxypivalinsäureneopentylglykolester durch basenkatalysierte Disproportionierung von Hydroxypivalinaldehyd.

Erdalkalimetallhydroxide sind nach der Lehre der DE-A 16 43 650 als Katalysatoren für die Reaktion von Hydroxypivalinaldehyd zu Hydroxypivalinsäureneopentylglykolester bekannt. Auch Erdalkalimetalloxide sind nach der DE-A 22 34 110 für diesen Zweck geeignet.

In der technischen Ausgestaltung liegt ein Nachteil dieser Katalysatoren darin, daß sie als Feststoffe dosiert werden müssen. Dies erfordert vor allem bei kontinuierlichem Betrieb einen erheblichen technischen Aufwand.

Mit Natrium- oder Kaliumhydroxid als Katalysator läuft die Reaktion unspezifisch ab; die Cannizzaro-Reaktion tritt als unerwünschte Nebenreaktion ein (Liebigs Ann. Chem. 1979, 1591-1601).

Der Erfindung lag somit die Aufgabe zugrunde, ein Verfahren zur Verfügung zu stellen, das ohne Feststoffdosierung des Katalysators auskommt.

Demgemäß wurde ein Verfahren zur Herstellung von Hydroxypivalinsäureneopentylglykolester durch basenkatalysierte Disproportionierung von Hydroxypivalinaldehyd gefunden, das dadurch gekennzeichnet ist, daß man die Reaktion in Gegenwart eines gut wasserlöslichen Erdalkalimetallsalzes und eines Alkalimetallhydroxids ausführt.

Die Reaktion läßt sich durch folgende Gleichung veranschaulichen: 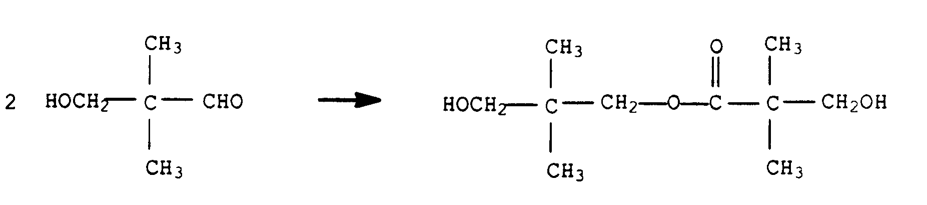
Der als Ausgangsstoff verwendete Hydroxypivalinaldehyd kann z.B. aus Isobutyraldehyd und wäßrigem Formaldehyd unter Trialkylaminkatalyse hergestellt werden (EP-A 0 173 976). Sowohl das Rohprodukt aus dieser Reaktion als auch das gereinigte Produkt sind als Ausgangsstoffe geeignet. Hydroxypivalinaldehyd wird üblicherweise mit einem Wassergehalt von 1 bis 50 Gew.-%, vorzugsweise 4 bis 20 Gew.-% eingesetzt.

Als Erdalkalimetallsalze kommen gut wasserlösliche Salze wie Halogenide oder Carbonsäuresalze sowie deren Hydrate in Frage. Gut wasserlöslich heißt, daß die Löslichkeit bei 25°C mindestens 10 g/l Wasser beträgt. Salze von Calcium, Strontium und Barium sind bevorzugt, von diesen besonders die Calciumsalze. Als Beispiele seien genannt: Calciumchlorid, Calciumbromid, Calciumiodid, Calciumformiat, Calciumacetat, Calciumpropionat, Calciumbutyrat, Calciumisobutyrat, Calcium-α-Methylbutyrat, Bariumchlorid, Bariumbromid, Bariumiodid, Bariumformiat, Bariumacetat, Bariumpropionat, Strontiumchlorid, Strontiumbromid, Strontiumiodid, Strontiumformiat, Strontiumacetat und Strontiumlactat.

Die Erdalkalimetallsalze können in einer Menge von 0,5 bis 15, vorzugsweise 0,5 bis 8 mol-%, bezogen auf Hydroxypivalinaldehyd, eingesetzt werden.

Sie werden bevorzugt in Form wäßriger Lösungen, besonders bevorzugt in Form konzentrierter wäßriger Lösungen zum Reaktionsansatz gegeben.

Als Alkalimetallhydroxide kommen die Hydroxide von Lithium, Natrium und Kalium in Betracht, wobei Natriumhydroxid bevorzugt ist. Man verwendet im allgemeinen 1 bis 3 mol Alkalihydroxid, vorzugsweise 1,8 bis 2,5 mol je mol Erdalkalimetallsalz. Die Alkalimetallhydroxide werden ebenfalls bevorzugt als konzentrierte wäßrige Lösungen eingesetzt, können aber auch als verdünnte Lösungen zur Anwendung kommen.

Eine mögliche Ausführungsform der Erfindung ist die Zugabe des Alkalimetallhydroxids zu einer Lösung, die Hydroxypivalinaldehyd und ein gut wasserlösliches Calciumsalz enthält. Im umgekehrten Fall, also bei der Zugabe eines gut wasserlöslichen Calciumsalzes zu einer Lösung, die Hydroxypivalinaldehyd und ein Alkalimetallhydroxid enthält, kann es zur Bildung unerwünschter Nebenprodukte kommen. Besonders für kontinuierliche Verfahren hat es sich als besonders vorteilhaft erwiesen, jeweils Lösungen der beiden Katalysatorkomponenten getrennt zum Hydroxypivalinaldehyd zuzusetzen.

Die Umsetzung erfolgt diskontinuierlich oder kontinuierlich, vorzugsweise kontinuierlich. Im Falle der kontinuierlichen Fahrweise kommen übliche Reaktoren wie Rohrreaktoren, Rührkesselkaskaden und Mehrkammer-Blasensäulen in Betracht. Besonders gute Ergebnisse werden mit Mehrkammer-Blasensäulen erzielt.

Die Reaktion wird vorzugsweise bei 40 bis 100°C, besonders bei 60 bis 80°C durchgeführt. Da der Druck keinen erkennbaren Einfluß auf das Verfahren hat, arbeitet man vorzugsweise bei Atmosphärendruck.

Unter den angegebenen Bedingungen beträgt die Reaktionszeit für einen nahezu vollständigen Umsatz im Falle der kontinuierlichen Arbeitsweise etwa 2 bis 60, in der Regel 5 bis 25 Minuten.

Der schwach basische Reaktoraustrag kann dann nach bekannten Methoden (z.B. EP-A 0 173 976) auf das Produkt aufgearbeitet oder unmittelbar für weitere Umsetzungen verwendet werden. So wird der schwach basische Reaktionsaustrag vorzugsweise mit 0,5 bis 2 mol einer Säure (bezogen auf 1 mol basische Katalysatorkomponente) versetzt, so daß das Reaktionsgemisch schwach sauer oder vorzugsweise neutral reagiert, und bei 50 bis 100°C mit Wasser extrahiert.

Die Isolierung des Hydroxypivalinsäureneopentylglykolesters geschieht nach üblichen Methoden destillativ.

Das erfindungsgemäße Verfahren gestattet die Herstellung von Hydroxypivalinsäureneopentylglykolester aus Hydroxypivalinaldehyd in hoher Ausbeute mit dem verfahrenstechnischen Vorteil, daß nur Lösungen dosiert zu werden brauchen.

Hydroxypivalinsäureneopentylglykolester ist ein wertvoller Ausgangsstoff für die Herstellung von Polyestern, Kunstharzen und Weichmachern.

### Beispiele

### Beispiel 1

Durch einen ölmantelbeheizten Mehrkammer-Blasensäulenreaktor (Volumen = 251 ml; Länge = 80 cm; Durchmesser = 2 cm; 9 Lochböden im Abstand von jeweils 8 cm; 3 Löcher mit einem Durchmesser von 3 mm je Boden) wurden in Sumpffahrweise bei 70°C pro Stunde 800 ml Hydroxypivalinaldehyd (HPA) mit einem Wassergehalt von 5 % (7,3 mol HPA), 171 ml einer 22 gew.-%igen wäßrigen Calciumacetat-Lösung (3,3 mol-% Calciumacetat bezogen auf HPA) sowie 36 mi einer 40 gew.-%igen wäßrigen NaOH-Lösung (6,6 mol-% NaOH, bezogen auf HPA) geleitet. Dies entspricht einer Verweilzeit von 15 min sowie einem Gesamtwassergehalt des Reaktionsgemisches von 21,2 %. Zur intensiven Durchmischung des Blasensäuleninhalts wurde zusätzlich ein Stickstoffstrom von 17 l/h eingespeist. Der Reaktoraustrag wurde mit 2,1 g konz. Ameisensäure je 100 g Austrag neutralisiert, wonach die Phasen bei 70°C getrennt wurden und das Produkt durch Destillation isoliert wurde.

Die Ausbeute an Hydroxypivalinsäureneopentylglykolester betrug 91 %.

### Beispiel 2

Durch einen Mehrkammer-Blasensäulenreaktor wie in Beispiel 1 wurden in Sumpffahrweise bei 70°C pro Stunde 928 ml Hydroxypivalinaldehyd mit einem Wassergehalt von 17 % (6,9 mol HPA), 45,9 mi einer 34 gew.-%igen wäßrigen CaCl₂-Lösung (2,7 mol-% CaCl₂ bezogen auf HPA) sowie 31,2 ml einer 40 gew.-%igen wäßrigen NaOH-Lösung (5,8 mol-% NaOH, bezogen auf HPA) geleitet. Dies entspricht einer Verweilzeit von 15 min sowie einem Gesamtwassergehalt des Reaktionsgemisches von 22 %. Zur intensiven Durchmischung des Blasensäuleninhalts wurde zusätzlich ein Stickstoffstrom von 17 l/h eingespeist. Der Reaktoraustrag wurde mit 2,0 g konz. Ameisensäure je 100 g Austrag neutralisiert, wonach die Phasen bei 70°C getrennt wurden und das Produkt durch Destillation isoliert wurde.

Die Ausbeute an Hydroxypivalinsäureneopentylglykolester betrug 92 %.

## Patentansprüche

1. Verfahren zur Herstellung von Hydroxypivalinsäureneopentylglykolester durch basenkatalysierte Disproportionierung von Hydroxypivalinaldehyd, dadurch gekennzeichnet, daß man die Reaktion in Gegenwart eines gut wasserlöslichen Erdalkalimetallsalzes und eines Alkalimetallhydroxids ausführt.

2. Verfahren nach Anspruch 1, dadurch gekennzeichnet, daß als Alkalihydroxid Natrium- oder Kaliumhydroxid verwendet wird.

3. Verfahren nach Anspruch 1 oder 2, dadurch gekennzeichnet, daß man bei kontinuierlicher Reaktionsführung jeweils eine wäßrige Lösung eines gut wasserlöslichen Erdalkalimetallsalzes und eines Alkalimetallhydroxids zum Hydroxypivalinaldehyd zusetzt.

## Claims

1. A process for preparing neopentyl glycol hydroxypivalate by base-catalyzed disproportionation of hydroxypivalaldehyde, wherein the reaction is carried out in the presence of a readily water-soluble alkaline earth metal salt and of an alkali metal hydroxide.

2. A process as claimed in claim 1, wherein sodium or potassium hydroxide is used as alkali metal hydroxide.

3. A process as claimed in claim 1 or 2, wherein in a continuous reaction an aqueous solution of a readily water-soluble alkaline earth metal salt and an aqueous solution of an alkali metal hydroxide are added to the hydroxypivalaldehyde.

## Revendications

1. Procédé de préparation de l'hydroxypivalate de néopentylglycol par la dismutation de l'hydroxypivalinaldéhyde catalysée par des bases, caractérisé en ce que l'on entreprend la réaction en présence d'un sel de métal alcalino-terreux parfaitement soluble dans l'eau et d'un hydroxyde de métal alcalin.

2. Procédé suivant la revendication 1, caractérisé en ce que l'on utilise l'hydroxyde de sodium ou l'hydroxyde de potassium à titre d'hydroxyde de métal alcalin.

3. Procédé suivant la revendication 1 ou 2, caractérisé en ce que lorsque l'on conduit la réaction en continu, on ajoute, à chaque fois, une solution aqueuse d'un sel de métal alcalino-terreux parfaitement soluble dans l'eau et un hydroxyde de métal alcalin à l'hydroxypivalinaldéhyde.
